# EUROPEAN PATENT APPLICATION

(11) **EP 4 512 352 A1**
(43) Date of publication of application: **26.02.2025**
(21) Application number: 24195678.8
(22) Date of filing: 21.08.2024
(51) Int. Cl.: A61B 17/80

(54) **PERIPROSTHETIC PLATE**

(30) Priority: 24.08.2023 US 202318454870
(71) Applicant: Globus Medical, Inc, Audubon, PA 19403 (US)
(72) Inventor: KEDZIERSKA, Anna, Philadelphia, 19128 (US); GRAY, Rebecca, Spring City, 19475 (US); BORDEAUX, Jean-Noel, West Chester, 19382 (US); MITCHELL, Madeline, Norristown, 19401 (US)
(74) Representative: Morabito, Sara

(57) **Abstract**

A periprosthetic plate and method of use are disclosed that includes a proximal segment having a top portion and an end portion with at least two curved hooks that are capable of passing through a patient's abductor muscles and rest medially on a tip of a patient's greater trochanter (GT), wherein the proximal segment includes a plurality of recessed bone screw holes. A periprosthetic proximal femur plate is disclosed that includes a head portion having at least six first polyaxial screw holes and a shaft portion connected to the shaft portion having a plurality of rows of at least two second polyaxial screw holes located at an angle from the horizontal from about thirty degrees to about sixty degrees. A combination periprosthetic proximal femur plate and greater trochanter (GT) plate construct is disclosed that includes a greater trochanter (GT) plate located on top of the periprosthetic proximal femur plate.

## Description

### TECHNICAL FIELD

The present disclosure generally relates to a plate to treat a long bone fracture located near an existing prosthesis that is typically at the distal or proximal femur.

### BACKGROUND

A periprosthetic fracture is a long bone fracture that occurs adjacent or around an existing prosthesis, typically at the distal or proximal femur. Due to an aging population, the frequency of these types of fractures is increasing.

A specialized implant for the proximal femur is required to treat Vancouver Type Ag, Ag + B, and B fractures. A Vancouver Ag fracture is characterized by a fracture of the Greater Trochanter, and a Vancouver B fracture is characterized by a fracture of the diaphysis right around the distal tip of a prosthesis such as a hip stem. Treatment methods for a Vancouver B fracture include a periprosthetic proximal femur plate, whereas treatment methods for a Vancouver Ag +B or a Vancouver Ag fracture include a greater trochanter ("GT") plate attached to a periprosthetic proximal femur plate. A greater trochanter ("GT") plate is only used in conjunction with a periprosthetic proximal femur plate, never on its own.

The current designs of periprosthetic proximal femur plates can cause soft tissue irritation due to the thickness of the head of the plate, which sits directly below the vastus ridge of the proximal femur. The attachment style of the current greater trochanter (GT) plate/ periprosthetic proximal femur plate constructs yields a similar concern. Overlap of the two plates near the vastus ridge area results in a prominent plate construct thickness which can cause soft tissue irritation.

Moreover, current designs of the greater trochanter (GT) plate include a closed ring construct that provides a suboptimal anatomical fit to the greater trochanter. The fixed geometry of the ring region as well as the challenge of fitting two plates to bone simultaneously, results in a poor fit.

A hook segment is a plate with hooks intended to go around the tip of the greater trochanter and is secured with screws. Current hook segment designs can have properties that are not ideal for this indication. This includes but is not limited to: 1) Too thick medial to lateral, which can cause patient discomfort; 2) insufficient screw options to properly secure the fracture fragments; and 3) screws not targeted to engage desired bony anatomy.

Thus, there exists a need in the art for a periprosthetic plate that is less intrusive to increase patient comfort, provides screw options to properly secure fracture fragments, and engages bony anatomy.

### SUMMARY

The following objects, features, advantages, aspects, and/or embodiments are not exhaustive and do not limit the overall disclosure. No single embodiment need provide each and every object, feature, or advantage. Any of the objects, features, advantages, aspects, and/or embodiments disclosed herein can be integrated with one another, either in full or in part.

It is a primary object, feature, and/or advantage of the present disclosure to improve on or overcome the deficiencies in the art.

It is a further object, feature, and/or advantage of the present disclosure is a periprosthetic plate that includes a proximal segment having a top portion and an end portion with at least two curved hooks that are capable of passing through a patient's abductor muscles and rest medially on the tip of a patient's greater trochanter (GT), wherein the proximal segment includes a plurality of recessed bone screw holes for attachment to a patient's femur with bone screws.

It is a further object, feature, and/or advantage of the present disclosure is the proximal segment includes a hook portion having at least two curved hooks and an attachment portion.

A further object, feature, and/or advantage of the present disclosure is that the plurality of recessed bone screw holes are recessed so that any inserted bone screws do not extend above the top portion of the proximal segment.

An aspect of the present disclosure is that the plurality of recessed bone screw holes are in sets of two recessed bone screw holes parallel to each other.

Another aspect of the present disclosure is a distal segment or shaft that is attached to or is integral with the proximal segment.

Yet another aspect of the present disclosure is the proximal segment includes a hook segment attached to at least two curved hooks and an attachment segment.

Another feature of the present disclosure is the hook segment includes a first plurality of recessed bone screw holes, the attachment segment includes a second plurality of recessed bone screw holes, and the distal segment or shaft includes a third plurality of recessed bone screw holes.

Yet another aspect of the present disclosure is wherein the first plurality of recessed bone screw holes are in sets of two and parallel to each other, the second plurality of recessed bone screw holes are in sets of two and parallel to each other, and the third plurality of recessed bone screw holes are staggered from side-to-side along the longitudinal length of the distal segment or shaft.

Still, yet another feature of the present disclosure is the first plurality of recessed bone screw holes, the second plurality of recessed bone screw holes, and the third plurality of recessed bone screw holes are recessed so that any inserted bone screws do not extend above the top portion of the proximal segment.

Another feature of the present disclosure is the proximal segment includes at least one cerclage cable hole set that can receive at least one cerclage cable.

Still, another aspect of the present disclosure is at least one cerclage crimp located in an opening in the proximal segment.

A further feature of the disclosure is the proximal segment includes at least one cerclage cable set that can each receive a cerclage cable and at least one cerclage crimp located in at least one opening in the proximal segment.

Still, another feature of the present disclosure is a periprosthetic plate that includes a hook segment having a top portion and an end portion with at least two curved hooks that are capable of passing through a patient's abductor muscles and rest medially on a tip of a patient's greater trochanter (GT), wherein the hook segment includes a first plurality of recessed bone screw holes that are recessed so that any inserted bone screws do not extend above the top portion of the hook segment and are in sets of two recessed bone screw holes that are parallel to each other, and an attachment segment that is attached or integral to the hook segment and includes a second plurality of recessed bone screw holes that are in sets of two and parallel to each other and beveled so that any inserted bone screws do not extend above the top portion of the hook segment and are in sets of two recessed bone screw holes that are parallel to each other.

Still, yet another feature of the present disclosure is a distal segment or shaft that is attached to or integral with the attachment segment and includes a third plurality of recessed bone screw holes that are staggered from side-to-side along the length of the distal segment or shaft, wherein the third plurality of recessed bone screw holes are beveled so that any inserted bone screws do not extend above the top portion of the proximal segment.

Another aspect of the present disclosure is a method utilizing a periprosthetic plate that includes securing a proximal segment into a patient having a top portion and an end portion with at least two curved hooks through a patient's abductor muscles that rest medially on a tip of a patient's greater trochanter (GT) by inserting bone screws into a first plurality of recessed bone screw holes but avoiding existing prosthesis or intramedullary nail, wherein the inserted bone screws do not extend above the top portion of the proximal segment.

An additional feature of the disclosure is a method for securing a distal segment or shaft that is attached to or integral with the proximal segment by inserting bone screws into a second plurality of recessed bone screw holes wherein the inserted bone screws do not extend above the top portion of the distal segment or shaft.

Yet another feature of the method of the present disclosure is inserting bone screws into the second plurality of recessed bone screw holes that are staggered from side-to-side along the length of the distal segment or shaft.

It is still yet another feature of the method of the present disclosure is inserting at least one cerclage cable through a plurality of holes in the proximal segment to secure the patient's bone.

It still another feature of the method of the present disclosure is applying tension to at least one cerclage cable and then securing the at least one cerclage cable with a cerclage crimp located in an opening within the proximal segment.

In still yet another aspect of the present disclosure is a periprosthetic proximal femur plate that includes a head portion having a plurality of first polyaxial screw holes, and a shaft portion, having a first lateral plate edge and second lateral plate edge, connected to the head portion having a plurality of rows of second polyaxial screw holes located at an angle from a first lateral plate edge and a second lateral plate edge of about thirty degrees to about seventy degrees.

It is a further object, feature, and/or advantage of the present disclosure is the first and second polyaxial screw holes have a cone of angulation in a range from about twenty-five degrees to about forty-five degrees.

Still another aspect of the present disclosure is wherein the first and second polyaxial screw holes have a cone of angulation in a range from about thirty degrees to about forty degrees.

An aspect of the present disclosure is the plurality of rows of the second polyaxial screw holes includes at least three rows.

Another aspect of the present disclosure is wherein the second polyaxial screw holes include a series of second polyaxial screw holes in a three-hole pattern.

Another feature of the present disclosure is the plurality of rows of polyaxial screw holes are located at an angle from the first lateral plate edge and the second lateral plate edge of about forty degrees to about sixty degrees.

Yet another aspect of the present disclosure is the plurality of rows of second polyaxial screw holes are located at an angle from the first lateral plate edge and the second lateral plate edge of about thirty-five degrees to about fifty-five degrees.

Still, yet another feature of the present disclosure is the head portion includes at least one orientation feature and at least one screw hole for connection with another plate.

Another feature of the present disclosure is a plurality of k-wire/suture openings.

Still another aspect of the present disclosure is at least one polyaxial screw hole for accepting an articulated tension device (ATD) located at the end of the shaft portion of the periprosthetic proximal femur plate.

A further feature of the disclosure is a combination periprosthetic proximal femur plate and greater trochanter (GT) plate construct that includes a periprosthetic proximal femur plate having a first lateral plate edge, a second lateral plate edge, and a head portion having a plurality of first polyaxial screw holes and a shaft portion connected to the head portion and having a plurality of rows of second polyaxial screw holes located at an angle from the first lateral plate edge and the second lateral plate edge of about thirty degrees to about seventy degrees, and a greater trochanter (GT) plate located on top of the periprosthetic proximal femur plate having a base portion with a plurality of third clearance screw holes that are aligned with the plurality of second polyaxial screw holes in the periprosthetic proximal femur plate and having a first arm and a second arm attached to the base portion and extending outward to wrap around a patient's greater trochanter (GT).

Still, another feature of the present disclosure is a portion of the periprosthetic proximal femur plate includes a portion of the periprosthetic proximal femur plate is uncovered from the greater trochanter (GT) plate, with at least one first polyaxial screw hole being visible or covered with no axial screw holes being visible.

Still, yet another feature of the present disclosure is a plurality of k-wire/suture openings in both the periprosthetic proximal femur plate and the greater trochanter (GT) plate.

Another aspect of the present disclosure is the first arm includes a plurality of fourth polyaxial screw holes, and the second arm includes a plurality of fifth polyaxial screw holes.

It is still yet another feature of the method of the present disclosure is a plurality of cylindrical recesses in the head portion of the periprosthetic proximal femur plate for receiving a plurality of downward cylindrical protrusions of the greater trochanter (GT) plate with a plurality of threaded openings in both the periprosthetic proximal femur plate and the greater trochanter (GT) plate located in the middle of the plurality of cylindrical protrusions to secure together with a plurality of threaded bolts.

Yet another aspect of the present disclosure includes at least one slot in the head portion of the periprosthetic proximal femur plate for receiving a downward protruding angled bar of the greater trochanter (GT) plate and a cylindrical recess in the head portion of the periprosthetic proximal femur plate for receiving a downward cylindrical protrusion with a threaded opening located in the middle of the cylindrical protrusion that can secure together the periprosthetic proximal femur plate and the greater trochanter (GT) plate with a threaded bolt.

It still another feature of the method of the present disclosure is of utilizing a combination periprosthetic proximal femur plate and greater trochanter (GT) plate construct, that includes inserting bone screws into a plurality of first polyaxial screw holes of a periprosthetic proximal femur plate having a head portion, a first lateral plate edge, and a second lateral plate edge, inserting bone screws into a plurality of rows of second polyaxial screw holes located in a shaft portion connected to the head portion, wherein the plurality of rows of second polyaxial screw holes located at an angle from the first lateral plate edge and the second lateral plate edge of about thirty degrees to about seventy degrees, and inserting bone screws into a plurality of third clearance screw holes located in a base portion of a greater trochanter (GT) plate located on top of the periprosthetic proximal femur plate, wherein the plurality of third clearance screw holes that are aligned with the plurality of second polyaxial screw holes in the periprosthetic proximal femur plate and having a first arm and a second arm attached to the base portion and extending outward to wrap around a patient's greater trochanter (GT).

It is yet another feature of the method of the present disclosure includes utilizing a combination periprosthetic proximal femur plate and greater trochanter (GT) plate construct and inserting an articulated tension device (ATD) located at an end of the shaft portion of the periprosthetic proximal femur plate.

These and/or other objects, features, advantages, aspects, and/or embodiments will become apparent to those skilled in the art after reviewing the following brief and detailed descriptions of the drawings. The present disclosure encompasses (a) combinations of disclosed aspects and/or embodiments and/or (b) reasonable modifications not shown or described.

### BRIEF DESCRIPTION OF THE DRAWINGS

Several embodiments in which the present disclosure can be practiced are illustrated and described in detail, wherein like reference characters represent like components throughout the several views. The drawings are presented for exemplary purposes and may not be to scale unless otherwise indicated.
**FIG. 1** shows a perspective view of a periprosthetic plate that includes a proximal segment, a distal segment or shaft, screw holes, and hooks.
**FIG. 2** is a bottom view of the periprosthetic plate of **FIG. 1****,** showing a proximal segment, a distal segment or shaft, screw holes, and hooks.
**FIG. 3** is top view of the proximal segment shown in **FIG. 1** without a distal segment or shaft.
**FIG. 4** is a bottom view of the proximal segment shown in **FIG. 3** without a distal segment or shaft.
**FIG. 5** is a perspective view of a periprosthetic plate mounted on a greater trochanter (GT) and close to a prosthesis.
**FIG. 6** is a perspective view of a periprosthetic plate with a cerclage crimp and cerclage cable holes.
**FIG. 7** is a perspective view of a periprosthetic plate mounted on a greater trochanter (GT) and secured with a cerclage cable.
**FIG. 8** shows a perspective view of a periprosthetic proximal femur plate mounted on a femur bone.
**FIG. 9** shows a top view of a periprosthetic proximal femur plate, including a head and a shaft.
**FIG. 10** shows a top view of a combination of the greater trochanter (GT) plate and periprosthetic proximal femur plate construct.
**FIG. 11** shows a side view of a combination of the periprosthetic proximal femur plate, and the greater trochanter (GT) shown in **FIG. 10****.**
**FIG. 12** shows a first embodiment of a connection mechanism for connecting the greater trochanter (GT) plate to the periprosthetic proximal femur plate.
**FIG. 13** shows a second embodiment of a connection mechanism for connecting the trochanter (GT) plate to the periprosthetic proximal femur plate.
**FIG. 14** shows a perspective view of greater trochanter (GT) plate cylindrical boss protrusions.
**FIG. 15** shows a side view of proximal femur plate cylindrical recesses that interlock with the cylindrical boss protrusions of **FIG. 14****.**
**FIG. 16** shows a third embodiment of a connection mechanism for connecting the trochanter (GT) plate to the periprosthetic proximal femur plate.
**FIG. 17** shows a top view of a threaded hole with a bolt in the greater trochanter (GT) plate for periprosthetic proximal femur plate connection.

An artisan of ordinary skill in the art need not view, within isolated figure(s), the near-infinite distinct combinations of features described in the following detailed description to facilitate an understanding of the present disclosure.

### DETAILED DESCRIPTION

The present disclosure is not to be limited to that described herein. Mechanical, electrical, chemical, procedural, and/or other changes can be made without departing from the spirit and scope of the present disclosure. Unless otherwise indicated, no features shown or described are essential to permit basic operation of the present disclosure.

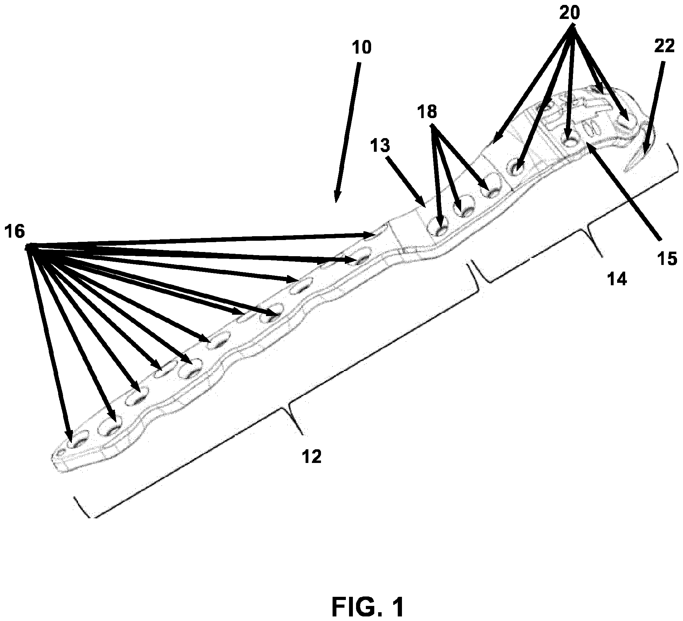

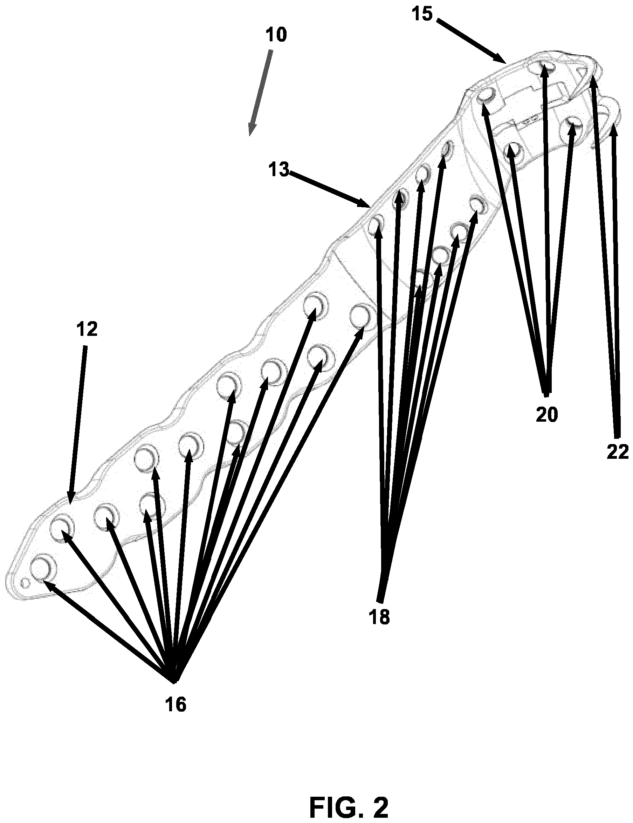

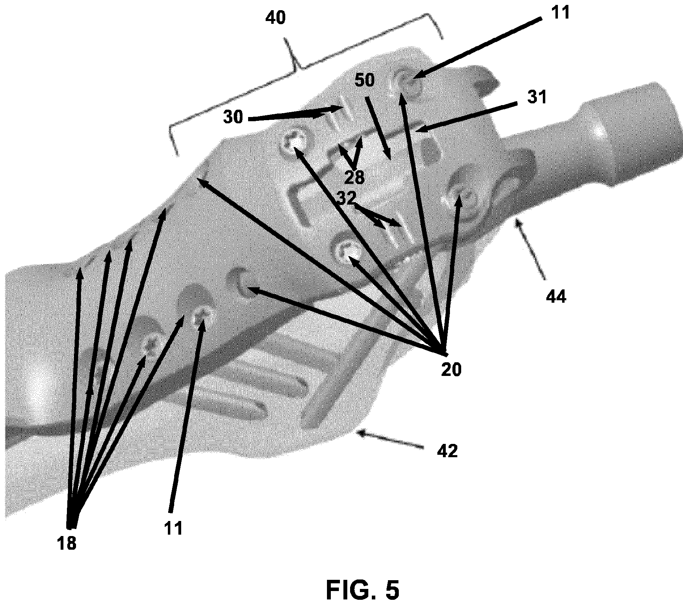

Referring now to FIGS. 1 and 2, a periprosthetic plate is generally indicated by the numeral 10. This periprosthetic plate 10 that includes a distal segment or shaft 12 and a proximal segment 14. This periprosthetic plate 10 may or may not include the distal segment or shaft 12. The version without a distal segment or shaft 12 is intended for simple Ag fractures. It will also be available in multiple sizes to match greater trochanter (GT) 40 anatomies (shown in FIG. 5) of a variety of patients. The periprosthetic plate 10 can have multiple lengths of the distal segment or shaft 12, with the longest able to span the entire femur bone of the patient.

The bone plates use biocompatible materials such as stainless steel (SS), cobalt base alloys, titanium alloys, bioceramics, composite materials, pure titanium, composite materials, and non-resorbable and bioresorbable polymers. These materials fall into a category such as bioinert, bioactive, bioresorbale, and porous. Typically, bioinert material is utilized for bone plates because if the bioactive material gets bonded to the bone or other soft tissues, there can be issues if bone plate removal or corrective surgery is required. The thickness of the bone plate depends on the specific material utilized and the type of patient bone, and potentially the specific anatomy of the patient.

The distal segment or shaft 12 includes holes 16 that are preferably beveled. The optimal depth of the distal segment or shaft 12 is only of a height that is slightly higher than the head of a bone screw 11, shown in FIG. 5, to minimize patient discomfort. Preferably, but not necessarily, the holes 16 are located in an alternating arrangement to minimize distance along the distal segment or shaft 12 that does not include a bone screw 11 and to ensure both sides of the distal segment or shaft 12 are secured to a bone.

Referring again to FIGS. 1 and 2, the proximal segment 14 includes an attachment segment 13 and a hook segment 15 connected together or preferably integral. There are a series of holes 18 for the attachment segment 13 that are preferably, but not necessarily, in parallel rows with the same preferred beveling to minimize any protrusion of a bone screw 11.

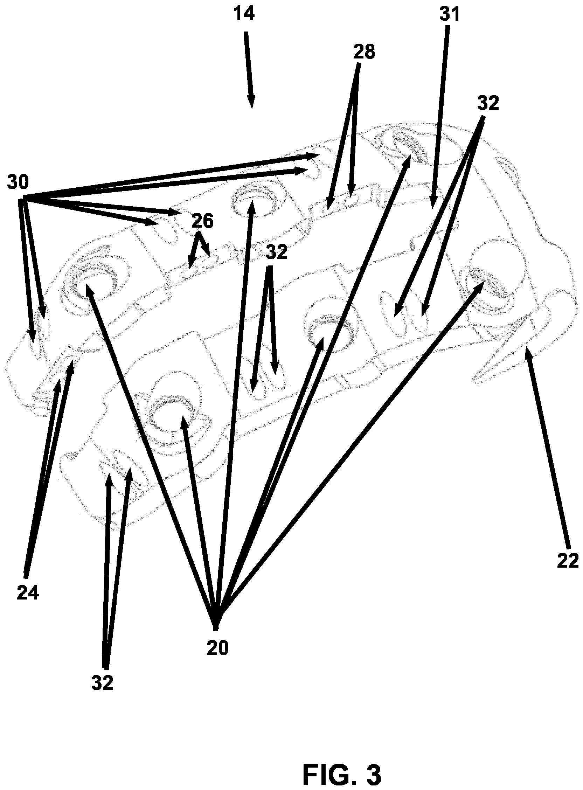

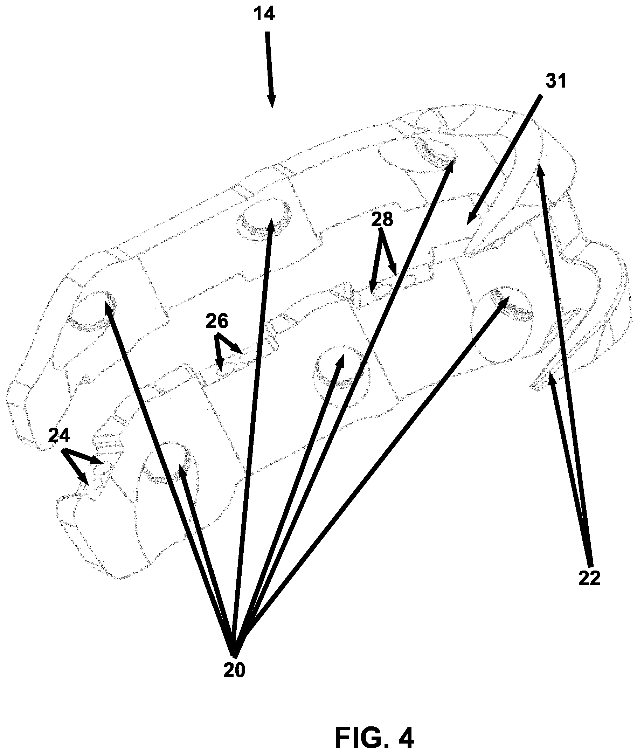

Referring now to FIGS. 3 and 4, hook segment 15 also includes a series of holes 20. The attachment segment 13, which preferably, but not necessarily, includes a series of holes 18 in parallel rows with the same preferred beveling to minimize any protrusion of a bone screw 11, as shown in FIG. 5. There are at least two hooks 22 to go around the tip of a greater trochanter (GT) 40 of a patient, and is secured with bone screws 11, shown in FIG. 5. The periprosthetic plate 10 may be positioned laterally or posterolaterally, with the hook(s) 22 passing through the abductor muscles of the patient and resting medially to the tip of the greater trochanter (GT) 40. If required, the periprosthetic plate 10 is tensioned to reduce a bone fracture. To secure the periprosthetic plate 10, screws are placed through holes 16, 18, and 20 in the periprosthetic plate 10 into the greater trochanter (GT) 40 of a patient.

Referring now to FIG. 5, the position of the periprosthetic plate 10 is shown in relation to the greater trochanter (GT) 40, lesser trochanter 42, and prosthesis 44. The bone screws 11 in the greater trochanter (GT) 40 are positioned to achieve two results. First, the bone screws 11 must not interfere with the existing prosthesis 44 or intramedullary nail (IM) (not shown), which passes through the greater trochanter (GT) 40 into the medullary canal.

Second, the nominal (co-axial with the hole) screw hole 16, 18, and 20 trajectories must target the areas of the proximal femur with the best (densest) bone. This bone is typically around the lesser trochanter (LT) 42; therefore, two posterior bone screws 11 are targeted to the lesser trochanter (LT) 42 area. The quality of the anterior bone of the greater trochanter (GT) 40 will vary according to the fracture and patient, but still requires the placement of bone screws 11. There is a minimum of four bone screws 11 in the greater trochanter (GT) 40 with two anterior and two posteriors.

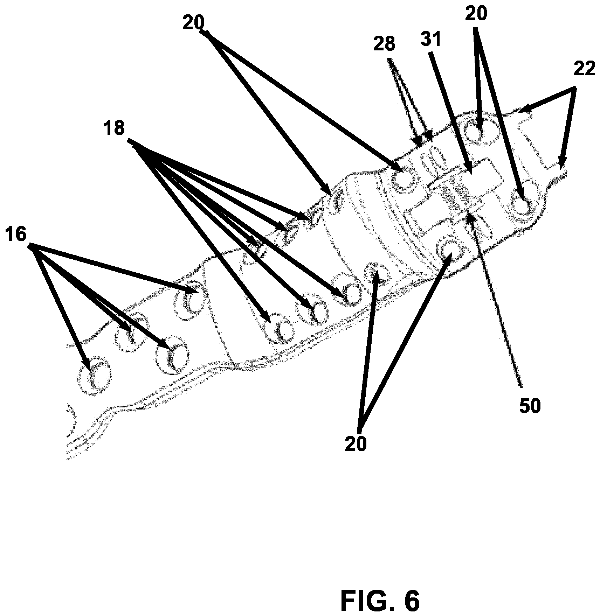

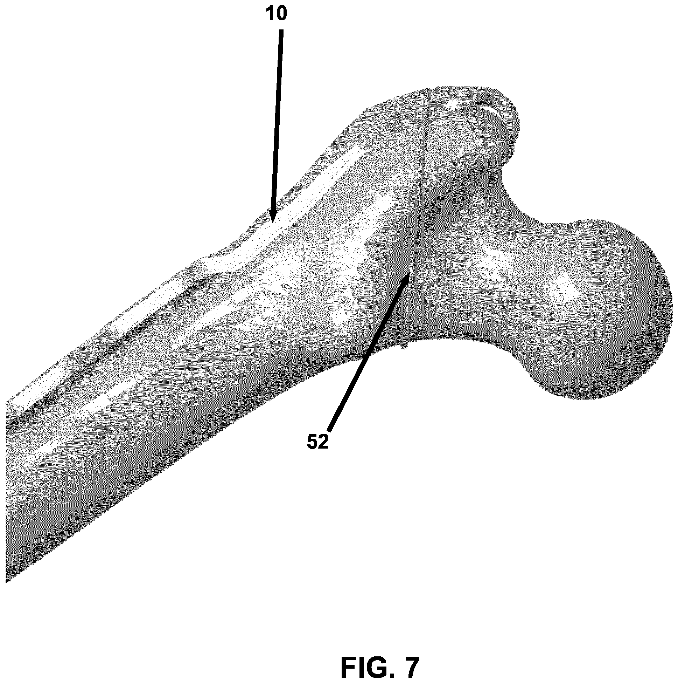

There is at least one cerclage cable hole set with four holes each. There is a first set of cable holes 24, a second set of cable holes 26, and a third set of cable holes 28 shown FIGS. 3 and 4. There are preferably, but not necessarily, a first pair of grooves 30 and a second pair of grooves 32 on each side of the opening in an opening in a center 31 of the top of the periprosthetic plate 10 that correspond to the first set of cable holes 24, the second set of cable holes 26 and a third set of cable holes 28. Therefore, the set of cable holes 24, 26, and 28 will each accommodate a single cerclage cable 52, shown in FIG. 7. At least one cable 52 may be passed through at least one cerclage crimp 50, which lay recessed along the center 31 of the top the periprosthetic plate 10, as shown in FIG. 6. Once tensioned, the at least one crimp 50 can be deformed to secure the at least one cable 52. A first pair of grooves 30 and a second pair of grooves 32 on each side of the opening in an opening in a center 31 of the top of the periprosthetic plate 10 could, in the alternative, also be grooves/recesses along the top (lateral side) of the periprosthetic plate 10 instead of holes thru the plate. Either are intended to maintain the position of the cerclage cable after tensioning.

Aspects of this technology disclosed include the thickness of the periprosthetic plate 10 across the lateral greater trochanter (GT) 40 is minimized to reduce prominence. The bone screw 11 trajectories are optimized to target the densest bone. The bone screws 11 are placed to avoid interference with the prosthesis 44. Moreover, hook segment 15 without the distal segment or shaft 12 may be optionally designed such that a portion of the hook segment 15 distal to the vastus ridge is not connected, as shown in FIGS. 3 and 4. Because they are not connected, the anterior, shown in FIG. 3, and posterior segments, shown in FIG. 4, of periprosthetic plate 10 can be individually contoured to match a patient's respective anatomies.

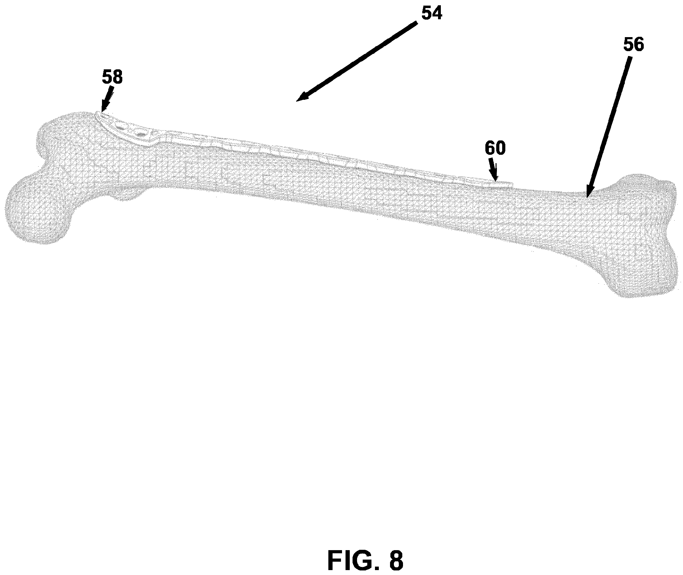

Another variation of a periprosthetic plate is a periprosthetic proximal femur plate, generally indicated by numeral 54 in FIG. 8, that is located on a patient's femur bone 56. The periprosthetic proximal femur plate 54 is designed to sit below the vastus ridge of the femur bone 56 and has a thinner chamfered proximal end 58 to reduce soft tissue irritation where the shaft of the periprosthetic proximal femur plate 54 gets gradually thinner in the distal portion 60. This thinner distal portion 60 allows for easier bending of the periprosthetic proximal femur plate 54 to better conform to the condyles when desired, which are the points of articulation and rotation at the end of the femur bone 56.

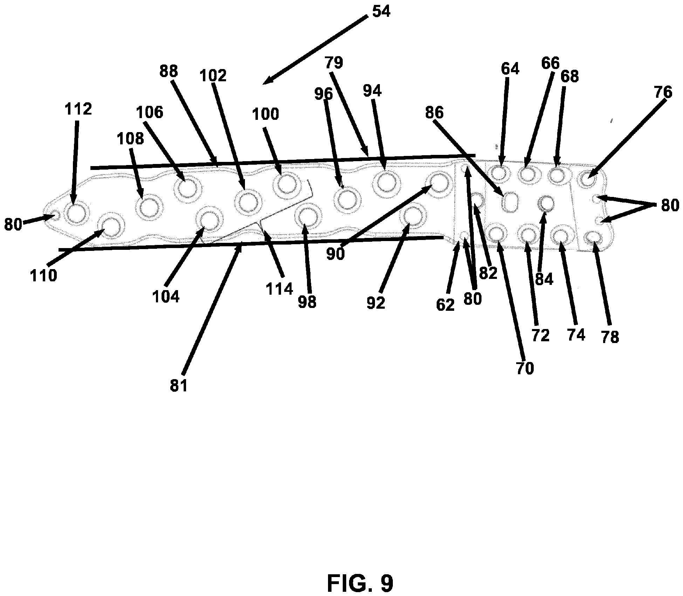

Referring now to FIG. 9, the periprosthetic proximal femur plate 54 is shown from a top view. The head of the periprosthetic proximal femur plate 54 is generally indicated by the numeral 62. Preferably, but not necessarily, the head 62 can include eight bone screw holes. This includes a first head bone screw hole 64, a second head bone screw hole 66, a third head bone screw hole 68, a fourth head bone screw hole 70, a fifth head bone screw hole 72, a sixth head bone screw hole 74, a seventh head bone screw hole 76, and an eighth head bone screw hole 78. Optimally, all of the head bone screw holes 62, 64, 66, 68, 70, 72, 74, 76, and 78 can be 3.5-millimeter screw holes with a forty-degree cone of angulation. An alternative embodiment is to have four to six of the head bone screw holes be 4.5-millimeter polyaxial screw holes with a thirty-degree cone of angulation with only the seventh head bone screw hole 76 and the eighth head bone screw hole 78 at the proximal end of the head 62 being 3.5-millimeter screw holes with a forty-degree cone of angulation. Optimally, the cone of angulation can range from zero to thirty degrees for a 4.5-millimeter polyaxial screw and zero to forty degrees, for a 3.5-millimeter polyaxial screw.

There is a first threaded hole for a greater trochanter (GT) plate connection 82 and a second threaded hole for a greater trochanter (GT) plate connection 84, and at least one orientation feature. There is also a slotted orientation feature for a greater trochanter (GT) plate 86 that can be a variety of shapes, preferably rectangular or circular. There are a series of k-wire/suture holes 80 at the proximal tip and along the sides of the periprosthetic proximal femur plate 54.

The shaft of the periprosthetic proximal femur plate 54 is generally indicated by the numeral 88. There is a first lateral plate edge 79 and a second lateral plate edge 81. The periprosthetic proximal femur plate 54 is just considered one length of plate 54. There will be multiple lengths available ranging from 119 millimeters to 388 millimeters in, at most around 35-millimeter increments. There are a variety of bone screw holes to be located with the shaft 88 in a variety of patterns. This includes a first-shaft bone screw hole 90, a second-shaft bone screw hole 92, a third-shaft bone screw hole 94, a fourth-shaft bone screw hole 96, a fifth-shaft bone screw hole 98, a sixth-shaft bone screw hole 100, a seventh shaft bone screw hole 102, an eighth shaft bone screw hole 104, a ninth shaft bone screw hole 106, a tenth shaft bone screw hole 108, and an eleventh shaft bone screw hole 110. The bone screw holes are staggered from about 4 millimeters to about 7 millimeters and preferably 5.50 millimeters apart across the width of the periprosthetic proximal femur plate 54 and spaced in a range from about ten 10 millimeters to about 15 millimeters and optimally 12.95 millimeters from one another down the periprosthetic proximal femur plate 54.

Preferably, the bone screw holes are at an angle of about twenty degrees to about 30 degrees and optimally about 23 degrees from the first lateral plate edge 79 and the second lateral plate edge 81 of the periprosthetic proximal femur plate 54.

Optimally, a three-hole pattern 114 is preferred at about forty-five degrees from the first lateral plate edge 79 and the second lateral plate edge 81 of shaft 88. These three-hole patterns allow for a screw fixation around an existing implant. The first shaft bone screw hole 90 and the second shaft bone screw hole 92 form a sequence of only a two-hole pattern and are located near the head 62 of the periprosthetic proximal femur plate 54. From there on, the third shaft bone screw hole 94, the fourth shaft bone screw hole 96, and the fifth shaft bone screw hole 98 form a first three-hole pattern. The sixth shaft bone screw hole 100, the seventh shaft bone screw hole 102, and the eighth shaft bone screw hole 104 form a second three-hole pattern, and the ninth shaft bone screw hole 106, the tenth shaft bone screw hole 108, and the eleventh shaft bone screw hole 110 form a third three-hole pattern. There is a shaft screw hole that accepts an articulated tension device (ATD) 112. Preferably, but not necessarily, this can be a 4.5-millimeter polyaxial screw hole with a thirty-degree cone of angulation.

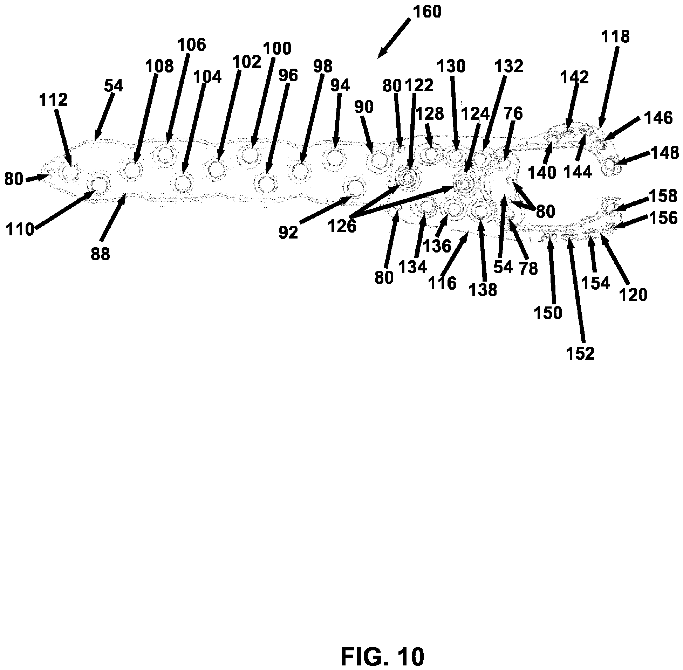

Referring now to FIG. 10, the periprosthetic proximal femur plate 54 can be combined with a greater trochanter (GT) plate 116 to form a combination construct 160. The proximal femur plate 54 and the greater trochanter (GT) plate 116 are connected by at least one threaded connection and preferably two, which includes a first threaded hole in the GT plate for periprosthetic proximal femur plate connection 122 and a second threaded hole in GT plate 124 for periprosthetic proximal femur plate connection that connects to the first threaded hole in periprosthetic proximal femur plate for GT plate connection 82 and the second threaded hole in periprosthetic proximal femur plate for GT plate connection 84.
The greater trochanter (GT) plate 116 has a first arm 116 and a second arm 120 that wraps around the greater trochanter region 40 extending around the anterior and posterior sides.

This combination construct 160 of proximal femur plate 54 and greater trochanter (GT) plate 116 provides significant advantages over the prior art that overlaps right below the vastus ridge where the plate overlap increases the construct 160 thickness and could cause the construct 160 to protrude above the vastus ridge which may increase the risk for soft tissue irritation and patient discomfort and potentially lead to revision surgery.

This combination construct 160 is designed to intentionally minimize plate thickness near the vastus ridge by avoiding overlap of the two plates, i.e., proximal femur plate 54 and greater trochanter (GT) plate 116, in this region. The seventh head bone screw hole 76 and the eighth head bone screw hole 78 in the proximal femur plate 54 are uncovered when the greater trochanter (GT) plate 116 is attached in order to reduce plate prominence in this area closest to the vastus ridge. A preferred design of the greater trochanter (GT) plate 116 includes ridges or eyebrows 126 for a smooth buildup around the first threaded hole in GT plate 116 for periprosthetic proximal femur plate connection 122 and around the second threaded hole in GT plate 116 for periprosthetic proximal femur plate connection 124 to help prevent soft tissue irritation from bolt heads protruding above the surface of the greater trochanter (GT) plate 116.

The first head bone screw hole in periprosthetic proximal femur plate 64 is aligned with the first head clearance screw hole in greater trochanter (GT) plate 128, the second head bone screw hole in periprosthetic proximal femur plate 66 is aligned with the second head clearance screw hole in greater trochanter (GT) plate 130, the third head bone screw hole in periprosthetic proximal femur plate 68 is aligned with the third head clearance screw hole in greater trochanter (GT) plate 132, the fourth head bone screw hole in periprosthetic proximal femur plate 70 is aligned with the fourth head clearance screw hole in greater trochanter (GT) plate 134, the fifth head bone screw hole in periprosthetic proximal femur plate 72 is aligned with the fifth head clearance screw hole in greater trochanter (GT) plate 136, and the sixth head bone screw hole in periprosthetic proximal femur plate 74 is aligned with the sixth head clearance screw hole in greater trochanter (GT) plate 138.

Preferably, but not necessarily, these six clearance holes in the greater trochanter (GT) plate 116, i.e., 128, 130. 132, 134, 136, and 138 correlate to the 3.5-millimeter polyaxial holes in the periprosthetic proximal femur plate 54, i.e., 64, 66, 68, 70, 72 and 74.

The first arm of the greater trochanter (GT) plate 118 and the second arm of the greater trochanter (GT) plate 120 wrap around the greater trochanter 40 and avoids the apex of the region as well as reduce soft tissue irritation with the first arm of the greater trochanter (GT) plate 118 includes a first polyaxial screw hole 140, a second polyaxial screw hole 142, a third polyaxial screw hole 144, a fourth polyaxial screw hole 146, and a fifth polyaxial screw hole 148. The second arm of the greater trochanter (GT) plate 120 includes a first polyaxial screw hole 150, a second polyaxial screw hole 152, a third polyaxial screw hole 154, a fourth polyaxial screw hole 156, and a fifth polyaxial screw hole 158.

The open geometry of the greater trochanter (GT) plate 116 provides for better conformance to the greater trochanter anatomy by allowing for bending. This provides a marked contrast to the prior art that involves a rigid, ring-like structure which makes it more difficult to conform to various greater trochanteric anatomies.

Some of the other features of the greater trochanter (GT) plate 116 include clearance holes for the k-wire/suture holes 80 that can also include locating the k-wire/suture holes 80 in between the screw holes 128, 130, 132, 134, 136, and 138 for suturing through the greater trochanter (GT) plate 116. Another feature is the seventh and eighth polyaxial screw holes 76 and 78 in the periprosthetic proximal femur plate 54. There are a number of screw holes in the first arm 118 and the second arm 120 of the trochanter (GT) plate, with preferably ten, i.e., 140, 142, 144, 146, 148, 150, 152, 154, 156, and 158, as described above, that provide numerous fixation options in the greater trochanter (GT) 40.

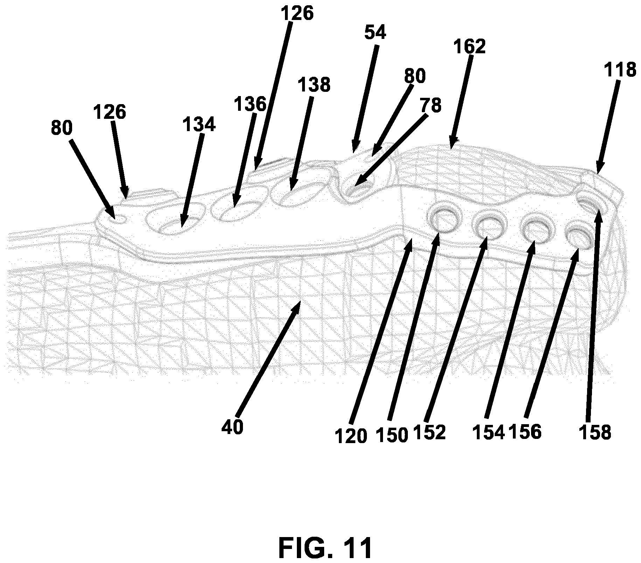

Referring now to FIG. 11, a side view of the greater trochanter (GT) 40 is shown, including the apex 162 of the greater trochanter (GT) 40. The ridges or eyebrows 126 on the top surface of the trochanter (GT) plate 40 are also shown, as well as the uncovered portion of the periprosthetic proximal femur plate 54.

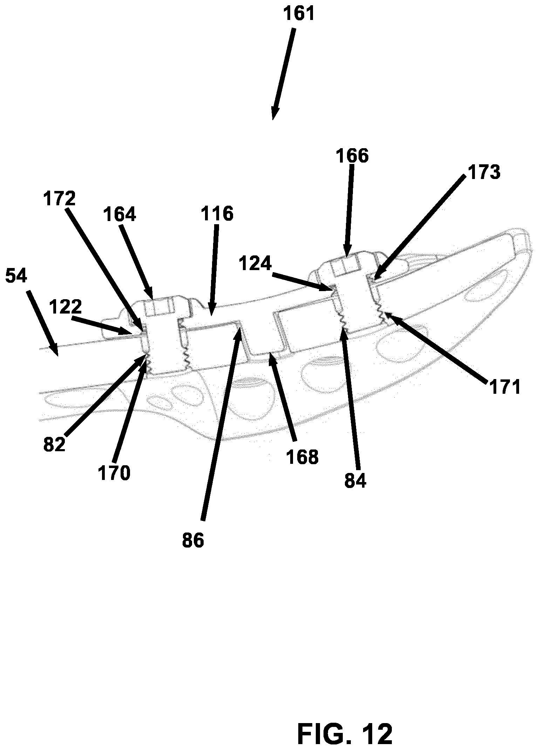

There are a variety of ways of connecting the trochanter (GT) plate 40 to the periprosthetic proximal femur plate 54. Referring now to FIG. 12, in a first embodiment that is generally indicated by the numeral 161, there are a first partially threaded bolt 164 and a second partially threaded bolt 166. There is a slanted rod 168 in the greater trochanter (GT) plate 116 that slides into the slotted orientation feature 86, shown in FIG. 9, in the periprosthetic proximal femur plate 54 for proper orientation. The slanted rod 168 is pointed upwards proximally in order to help resist the tension forces being exerted by the abductors. This offers a mechanical advantage in terms of the stability of the combination of greater trochanter (GT) plate and periprosthetic proximal femur plate construct 160. There are preferably two threaded connections in this first embodiment. There is a first partially threaded bolt 164 and a second partially threaded bolt 166 that can be received within the threads 172 and 173, respectively, located in the greater trochanter (GT) plate 116. Optionally, the threads 172 and 173 in the greater trochanter (GT) plate 116 allow the bolts, e.g., M5 X 0.8 bolts, to be pre-assembled and retained within the greater trochanter (GT) plate 116. The first partially threaded bolt 164 and a second partially threaded bolt 166 are partially threaded to allow the greater trochanter (GT) plate 116 and the threads 170 and171, respectively, in periprosthetic proximal femur plate 54 to be oriented properly prior to connecting them.

The first partially threaded bolt 164 is positioned within the first threaded hole in the periprosthetic proximal femur plate for the GT plate connection 82, and the first threaded hole in the GT plate for the periprosthetic proximal femur plate connection 122 with threads 170 in the periprosthetic proximal femur plate 54 and threads 172 in the greater trochanter (GT) plate 116. The second partially threaded bolt 166 is positioned within the second threaded hole in the periprosthetic proximal femur plate for GT plate connection 84, and the second threaded hole in the GT plate for periprosthetic proximal femur plate connection 124 with threads 171 in the periprosthetic proximal femur plate 54 and threads 173 in the greater trochanter (GT) plate 116.

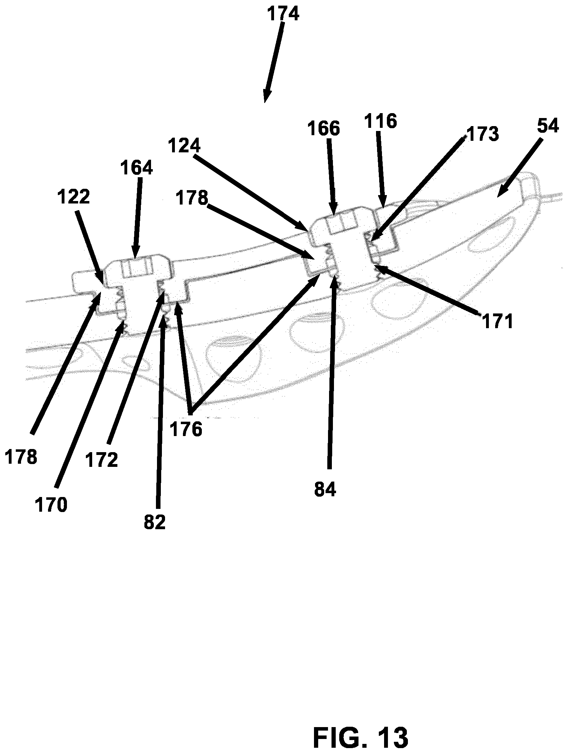

The second embodiment of connecting the trochanter (GT) plate to the periprosthetic proximal femur plate is generally indicated by numeral 174 in FIG. 13. There is the first partially threaded bolt 164 and the second partially threaded bolt 166 that can be received within the threads 172 and 173, respectively, located in the greater trochanter (GT) plate 116. Optionally, the threads 172 and 173 in the greater trochanter (GT) plate 116 allow the bolts, e.g., M5 X 0.8 bolts, to be pre-assembled and retained within the greater trochanter (GT) plate 116. The first partially threaded bolt 164 and a second partially threaded bolt 166 are partially threaded to allow the greater trochanter (GT) plate 116 and the threads 170 and 171, respectively, in periprosthetic proximal femur plate 54 to be oriented properly prior to connecting them.

The first partially threaded bolt 164 is positioned within the first threaded hole 170 in the periprosthetic proximal femur plate 82 and the first threaded hole in the GT plate for periprosthetic proximal femur plate connection 122 with threads 172. The second partially threaded bolt 166 is positioned within the second threaded hole 171 in the periprosthetic proximal femur plate 84 and the second threaded hole in the GT plate for periprosthetic proximal femur plate connection 124 with threads 173. Instead of an angled bar, the connection is made through greater trochanter (GT) plate cylindrical boss protrusions 178 that are located around the threaded connections 172 in the greater trochanter (GT) plate 116 that also function as orientation features. These cylindrical boss protrusions 178 on the underside of the greater trochanter (GT) plate 116 key into the cylindrical recesses 176 in the periprosthetic proximal femur plate 54. An advantage to this second embodiment 174 is that the heads of the partially threaded bolts 164 and 166 can be lower compared to the first embodiment 161.

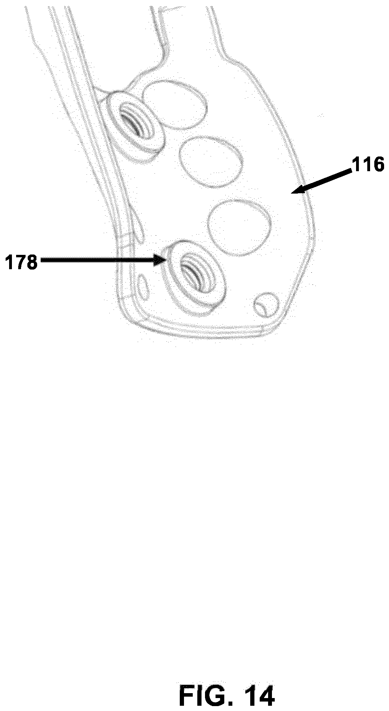

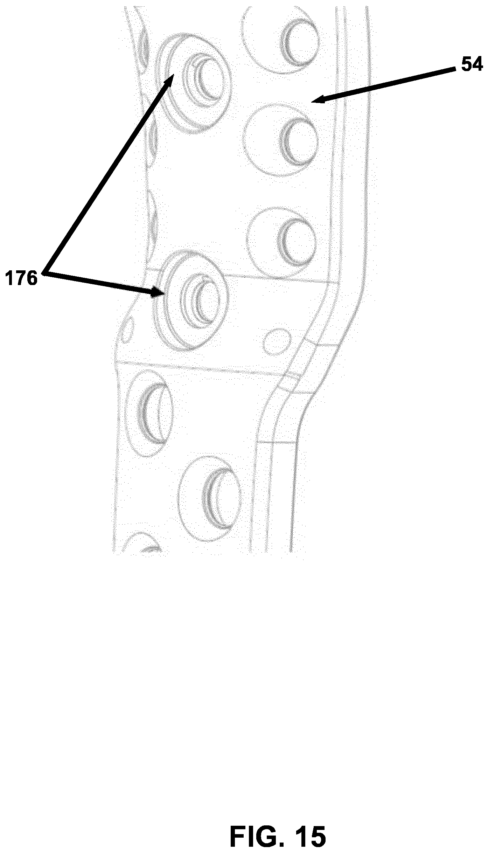

Referring now to FIG. 14, the cylindrical boss protrusions 178 on the underside of the greater trochanter (GT) plate 116 are more clearly shown. Referring now to FIG. 15, the cylindrical recesses 176 in the periprosthetic proximal femur plate 54 are more clearly shown.

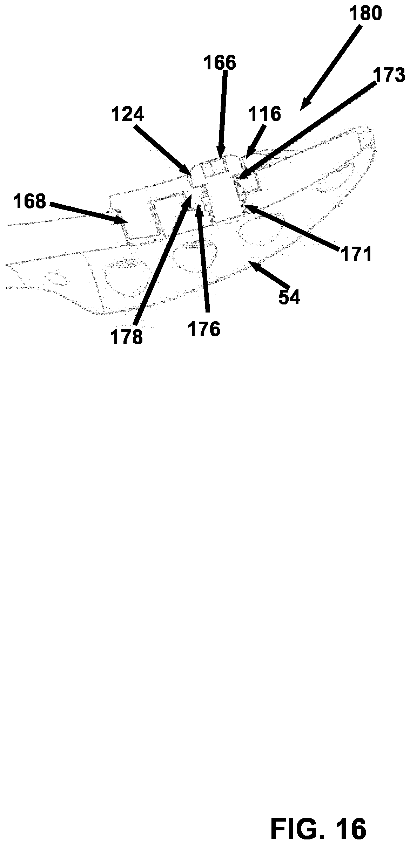

The third embodiment includes a slimmer profile than the first embodiment, and the second embodiment of connecting the trochanter (GT) plate 116 to the periprosthetic proximal femur plate 54 and is generally indicated by the numeral 180. This design reduces soft tissue irritation as much as possible. The connection mechanism has one threaded connection and two orientation features. Referring now to FIG. 16, there is the second partially threaded bolt 166 that can be received within the threads 173 located in the greater trochanter (GT) plate 116. Optionally, the threads 173 in the greater trochanter (GT) plate 116 allow the bolts, e.g., M5 X 0.8 bolts, to be pre-assembled and retained within the greater trochanter (GT) plate 116. This second partially threaded bolt 166 is partially threaded to allow the greater trochanter (GT) plate 116 and the threads 171 in periprosthetic proximal femur plate 54 to be oriented properly prior to connecting them.

The connection is made through greater trochanter (GT) plate cylindrical boss protrusions 178 that are located around the threaded connections 173 in the greater trochanter (GT) plate 116 that also function as orientation features. This cylindrical boss protrusion 178 on the underside of the greater trochanter (GT) plate 116 key into the cylindrical recess 176 in the periprosthetic proximal femur plate 54. There is a slanted rod 168 in the greater trochanter (GT) plate 116 that slides into the slotted orientation feature for GT plate 86, shown in FIG. 9, in the periprosthetic proximal femur plate 54 for proper orientation. The slanted rod 168 is pointed upwards proximally in order to help resist the tension forces being exerted by the abductors. This offers a mechanical advantage in terms of the stability of the combination of greater trochanter (GT) plate and periprosthetic proximal femur plate construct 160.

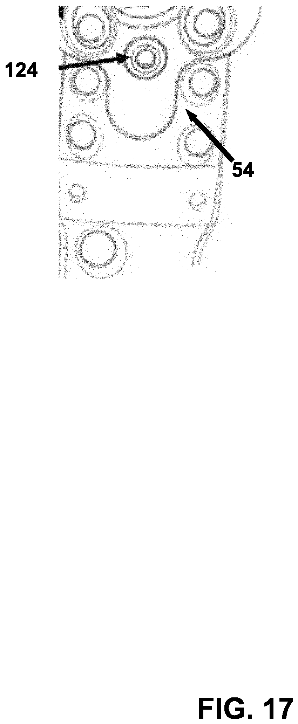

Referring now to FIG. 17, the plate bolt attachment hole 124 is shown in greater detail on the periprosthetic proximal femur plate 54.

From the foregoing, it can be seen that the present disclosure accomplishes at least all of the stated objectives.

### LIST OF REFERENCE CHARACTERS

The following table of reference characters and descriptors are not exhaustive, nor limiting, and include reasonable equivalents. If possible, elements identified by a reference character below and/or those elements which are near ubiquitous within the art can replace or supplement any element identified by another reference character.

**Table 1: List of Reference Characters**

| | |
|---|---|
| **10** | Periprosthetic plate |
| **11** | Bone screw |
| **12** | Distal segment or shaft |
| **13** | Attachment segment |
| **14** | Proximal segment |
| **15** | Hook segment |
| **16** | Holes for the distal plate or shaft |
| **18** | Holes for the attachment segment |
| **20** | Holes for the hook segment |
| **22** | Hooks |
| **24** | First set of cable holes |
| **26** | Second set of cable holes |
| **28** | Third set of cable holes |
| **30** | First pair of grooves |
| **31** | Opening in the center of the top of the periprosthetic plate |
| **32** | Second pair of grooves |
| **40** | Greater trochanter (GT) |
| **42** | Lesser trochanter |
| **44** | Prosthesis |
| **50** | Cerclage crimp |
| **52** | Cerclage cable |
| **54** | Periprosthetic proximal femur plate |
| **56** | Femur bone |
| **58** | Thinner chamfered proximal end portion of the periprosthetic proximal femur plate positioned below the vastus ridge. |
| **60** | Thinner portion of the periprosthetic proximal femur plate at a distal portion |
| **62** | Head of the periprosthetic proximal femur plate |
| **64** | First head bone screw hole in periprosthetic proximal femur plate |
| **66** | Second head bone screw hole in periprosthetic proximal femur plate |
| **68** | Third head bone screw hole in periprosthetic proximal femur plate |
| **70** | Fourth head bone screw hole in periprosthetic proximal femur plate |
| **72** | Fifth head bone screw hole in periprosthetic proximal femur plate |
| **74** | Sixth head bone screw hole in periprosthetic proximal femur plate |
| **76** | Seventh head bone screw hole in periprosthetic proximal femur plate |
| **78** | Eighth head bone screw hole in periprosthetic proximal femur plate |
| **79** | First lateral plate edge |
| **80** | K-wire/suture holes |
| **81** | Second lateral plate edge |
| **82** | First threaded hole in periprosthetic proximal femur plate for GT plate connection |
| **84** | Second threaded hole in periprosthetic proximal femur plate for GT plate connection |
| **86** | Slotted orientation feature for GT plate |
| **88** | Shaft of the periprosthetic proximal femur plate |
| **90** | First shaft bone screw hole |
| **92** | Second shaft bone screw hole |
| **94** | Third shaft bone screw hole |
| **96** | Fourth shaft bone screw hole |
| **98** | Fifth shaft bone screw hole |
| **100** | Sixth shaft bone screw hole |
| **102** | Seventh shaft bone screw hole |
| **104** | Eighth shaft bone screw hole |
| **106** | Ninth shaft bone screw hole |
| **108** | Tenth shaft bone screw hole |
| **110** | Eleventh shaft bone screw hole |
| **112** | Shaft screw hole that accepts ATD |
| **114** | Hole pattern, e.g., three-hole pattern |
| **116** | Greater trochanter (GT) plate |
| **118** | First arm of the greater trochanter (GT) plate |
| **120** | Second arm of the greater trochanter (GT) plate |
| **122** | First threaded hole in GT plate for periprosthetic proximal femur plate connection |
| **124** | Second threaded hole in GT plate for periprosthetic proximal femur plate connection |
| **126** | Ridges or eyebrows on the top surface of the GT plate |
| **128** | First head bone screw hole in greater trochanter (GT) plate |
| **130** | Second head bone screw hole in greater trochanter (GT) plate |
| **132** | Third head bone screw hole in greater trochanter (GT) plate |
| **134** | Fourth head bone screw hole in greater trochanter (GT) plate |
| **136** | Fifth head bone screw hole in greater trochanter (GT) plate |
| **138** | Sixth head bone screw hole in greater trochanter (GT) plate |
| **140** | First polyaxial screw hole of the first arm of the greater trochanter (GT) plate |
| **142** | Second polyaxial screw hole of the first arm of the greater trochanter (GT) plate |
| **144** | Third polyaxial screw hole of the first arm of the greater trochanter (GT) plate |
| **146** | Fourth polyaxial screw hole of the first arm of the greater trochanter (GT) plate |
| **148** | Fifth polyaxial screw hole of the first arm of the greater trochanter (GT) plate |
| **150** | First polyaxial screw hole of the second arm of the greater trochanter (GT) plate |
| **152** | Second polyaxial screw hole of the second arm of the greater trochanter (GT) plate |
| **154** | Third polyaxial screw hole of the second arm of the greater trochanter (GT) plate |
| **156** | Fourth polyaxial screw hole of the second arm of the greater trochanter (GT) plate |
| **158** | Fifth polyaxial screw hole of the second arm of the greater trochanter (GT) plate |
| **160** | Combination of greater trochanter (GT) plate and periprosthetic proximal femur plate construct |
| **161** | First embodiment of a connection mechanism for connecting the trochanter (GT) plate to the periprosthetic proximal femur plate |
| **162** | Apex |
| **164** | First partially threaded bolt |
| **166** | Second partially threaded bolt |
| **168** | Slanted rod |
| **170** | Threads in the periprosthetic proximal femur plate for the first partially threaded bolt |
| **171** | Threads in the periprosthetic proximal femur plate for the second partially threaded bolt |
| **172** | Threads in the greater trochanter (GT) plate for the first partially threaded bolt |
| **173** | Threads in the greater trochanter (GT) plate for the second partially threaded bolt |
| **174** | Second embodiment of a connection mechanism for connecting the trochanter (GT) plate to the periprosthetic proximal femur plate |
| **176** | Proximal femur plate cylindrical recesses |
| **178** | Greater trochanter (GT) plate cylindrical boss protrusions |
| **180** | Third embodiment of a connection mechanism for connecting the trochanter (GT) plate to the periprosthetic proximal femur plate |

### GLOSSARY

Unless defined otherwise, all technical and scientific terms used above have the same meaning as commonly understood by one of ordinary skill in the art to which embodiments of the present disclosure pertain.

The terms "a," "an," and "the" include both singular and plural referents.

The term "or" is synonymous with "and/or" and means any one member or combination of members of a particular list.

As used herein, the term "exemplary" refers to an example, an instance, or an illustration, and does not indicate a most preferred embodiment unless otherwise stated.

The term "about" as used herein refers to slight variations in numerical quantities with respect to any quantifiable variable. Inadvertent error can occur, for example, through use of typical measuring techniques or equipment or from differences in the manufacture, source, or purity of components.

The term "substantially" refers to a great or significant extent. "Substantially" can thus refer to a plurality, majority, and/or a supermajority of said quantifiable variables, given proper context.

The term "generally" encompasses both "about" and "substantially."

The term "configured" describes structure capable of performing a task or adopting a particular configuration. The term "configured" can be used interchangeably with other similar phrases, such as constructed, arranged, adapted, manufactured, and the like.

Terms characterizing sequential order, a position, and/or an orientation are not limiting and are only referenced according to the views presented.

The "invention" is not intended to refer to any single embodiment of the particular invention but encompass all possible embodiments as described in the specification and the claims. The "scope" of the present disclosure is defined by the appended claims, along with the full scope of equivalents to which such claims are entitled. The scope of the disclosure is further qualified as including any possible modification to any of the aspects and/or embodiments disclosed herein which would result in other embodiments, combinations, subcombinations, or the like that would be obvious to those skilled in the art.

The invention could be defined inter alia by the following examples:
1. A periprosthetic plate, comprising: a proximal segment having a top portion and an end portion with at least two curved hooks that are capable of passing through a patient's abductor muscles and rest medially on a tip of a patient's greater trochanter (GT), wherein the proximal segment includes a plurality of recessed bone screw holes for attachment to a patient's femur with bone screws.
2. The periprosthetic plate according to Example 1, wherein the proximal segment includes a hook portion having the at least two curved hooks and an attachment portion.
3. The periprosthetic plate according to Example 1, wherein the plurality of recessed bone screw holes are beveled so that any inserted bone screws do not extend above the top portion of the proximal segment.
4. The periprosthetic plate according to Example 1, wherein the plurality of recessed bone screw holes are in sets of two recessed bone screw holes that are parallel to each other.
5. The periprosthetic plate according to Example 4, wherein the plurality of recessed bone screw holes are in two sets of at least two recessed bone screw holes that are parallel to each other.
6. The periprosthetic plate according to Example 1, further comprising a distal segment or shaft that is attached to or is integral with the proximal segment.
7. The periprosthetic plate according to Example 6, wherein the proximal segment includes a hook segment attached to the at least two curved hooks and an attachment segment.
8. The periprosthetic plate according to Example 7, wherein the hook segment includes a first plurality of recessed bone screw holes, the attachment segment includes a second plurality of recessed bone screw holes, and the distal segment or shaft includes a third plurality of recessed bone screw holes.
9. The periprosthetic plate according to Example 8, wherein the first plurality of recessed bone screw holes are in sets of two and parallel to each other, the second plurality of recessed bone screw holes are in sets of two and parallel to each other and the third plurality of recessed bone screw holes are staggered from side-to-side along the longitudinal length of the distal segment or shaft.
10. The periprosthetic plate according to Example 9, wherein the first plurality of recessed bone screw holes, the second plurality of recessed bone screw holes, and the third plurality of recessed bone screw holes are beveled so that any inserted bone screws do not extend above the top portion of the proximal segment.
11. The periprosthetic plate according to Example 1, wherein the proximal segment includes at least one cerclage cable hole set that can receive at least one cerclage cable.
12. The periprosthetic plate according to Example 11, further comprising at least one cerclage crimp located in an opening in the proximal segment.
13. The periprosthetic plate according to Example 1, wherein the proximal segment includes at least one cerclage cable set that can each receive a cerclage cable and at least one cerclage crimp located in at least one opening in the proximal segment.
14. A periprosthetic plate, comprising: a hook segment having a top portion and an end portion with at least two curved hooks that are capable of passing through a patient's abductor muscles and rest medially on a tip of a patient's greater trochanter (GT), wherein the hook segment includes a first plurality of recessed bone screw holes that are beveled so that any inserted bone screws do not extend above the top portion of the hook segment and are in sets of two recessed bone screw holes that are parallel to each other; and an attachment segment that is attached or integral to the hook segment and includes a second plurality of recessed bone screw holes that are in sets of two and parallel to each other and beveled so that any inserted bone screws do not extend above the top portion of the hook segment and are in sets of two recessed bone screw holes that are parallel to each other.
15. The periprosthetic plate according to Example 14, further comprising a distal segment or shaft that is attached to or integral with the attachment segment and includes a third plurality of recessed bone screw holes that are staggered from side-to-side along the length of the distal segment or shaft, wherein the third plurality of recessed bone screw holes are beveled so that any inserted bone screws do not extend above the top portion of the proximal segment.
16. A method utilizing a periprosthetic plate, comprising: securing a proximal segment into a patient having a top portion and an end portion with at least two curved hooks through a patient's abductor muscles that rest medially on a tip of a patient's greater trochanter (GT) by inserting bone screws into a first plurality of recessed bone screw holes but avoiding existing prosthesis or intramedullary nail, wherein the inserted bone screws do not extend above the top portion of the proximal segment.
17. The method utilizing a periprosthetic plate according to Example 16, further comprising securing a distal segment or shaft that is attached to or integral with the proximal segment by inserting bone screws into a second plurality of recessed bone screw holes wherein the inserted bone screws do not extend above the top portion of the distal segment or shaft.
18. The method utilizing a periprosthetic plate according to Example 17, further comprising inserting bone screws into the second plurality of recessed bone screw holes that are staggered from side-to-side along the length of the distal segment or shaft.
19. The method utilizing a periprosthetic plate according to Example 16, further comprising inserting at least one cerclage cable through a plurality of holes in the proximal segment to secure the patient's bone.
20. The method utilizing a periprosthetic plate according to Example 19, further comprising applying tension to the at least one cerclage cable and then securing the at least one cerclage cable with a cerclage crimp located in an opening within the proximal segment.

## Claims

1. A periprosthetic plate, comprising: a proximal segment having a top portion and an end portion with at least two curved hooks that are capable of passing through a patient's abductor muscles and rest medially on a tip of a patient's greater trochanter (GT), wherein the proximal segment includes a plurality of recessed bone screw holes for attachment to a patient's femur with bone screws.

2. The periprosthetic plate according to Claim 1, wherein the proximal segment includes a hook portion having the at least two curved hooks and an attachment portion.

3. The periprosthetic plate according to any one of the preceding claims, wherein the plurality of recessed bone screw holes are beveled so that any inserted bone screws do not extend above the top portion of the proximal segment.

4. The periprosthetic plate according to any one of the preceding claims, wherein the plurality of recessed bone screw holes are in sets of two recessed bone screw holes that are parallel to each other.

5. The periprosthetic plate according to Claim 4, wherein the plurality of recessed bone screw holes are in two sets of at least two recessed bone screw holes that are parallel to each other.

6. The periprosthetic plate according to any one of the preceding claims, further comprising a distal segment or shaft that is attached to or is integral with the proximal segment.

7. The periprosthetic plate according to Claim 6, wherein the proximal segment includes a hook segment attached to the at least two curved hooks and an attachment segment.

8. The periprosthetic plate according to Claim 7, wherein the hook segment includes a first plurality of recessed bone screw holes, the attachment segment includes a second plurality of recessed bone screw holes, and the distal segment or shaft includes a third plurality of recessed bone screw holes.

9. The periprosthetic plate according to Claim 8, wherein the first plurality of recessed bone screw holes are in sets of two and parallel to each other, the second plurality of recessed bone screw holes are in sets of two and parallel to each other and the third plurality of recessed bone screw holes are staggered from side-to-side along the longitudinal length of the distal segment or shaft.

10. The periprosthetic plate according to Claim 9, wherein the first plurality of recessed bone screw holes, the second plurality of recessed bone screw holes, and the third plurality of recessed bone screw holes are beveled so that any inserted bone screws do not extend above the top portion of the proximal segment.

11. The periprosthetic plate according to any one of the preceding claims, wherein the proximal segment includes at least one cerclage cable hole set that can receive at least one cerclage cable.

12. The periprosthetic plate according to Claim 11, further comprising at least one cerclage crimp located in an opening in the proximal segment.

13. The periprosthetic plate according to any one of the preceding claims, wherein the proximal segment includes at least one cerclage cable set that can each receive a cerclage cable and at least one cerclage crimp located in at least one opening in the proximal segment.
